# EUROPEAN PATENT SPECIFICATION

(11) **EP 1 151 138 B1**
(45) Date of publication and mention of the grant of the patent: **29.12.2004**
(21) Application number: 00904696.2
(22) Date of filing: 01.02.2000
(51) Int. Cl.: C12Q 1/68

(54) **USE OF FLUORESCENT MOLECULAR BEACONS IN THE DETECTION OF METHYLATED NUCLEIC ACIDS**
VERWENDUNG VON FLUORESZIERENDEN "MOLECULAR BEACONS" ZUM NACHWEIS VON METHYLIERTEN NUKLEINSÄUREN
UTILISATION DE MARQUEURS MOLECULAIRES FLUORESCENTS DANS LA DETECTION D'ACIDES NUCLEIQUES METHYLES

(30) Priority: 01.02.1999 AU PP844899
(43) Date of publication of application: 07.11.2001
(73) Proprietor: Epigenomics AG, 10435 Berlin (DE)
(72) Inventor: KAY, Peter, H., Nedlands, W.A. 6009 (AU)
(74) Representative: Schubert, Klemens, Dr.
(86) International application number: PCT/AU2000/000053
(87) International publication number: WO 2000/046398

(56) References cited:
- WO-A-98/02449
- WO-A-98/26093
- GB-A- 2 333 596
- DAVIS R L ET AL: "EXPRESSION OF A SINGLE TRANSFECTED CDNA CONVERTS FIBROBLASTS TO MYOBLASTS" CELL, CELL PRESS, CAMBRIDGE, NA, US, vol. 51, 24 December 1987 (1987-12-24), pages 987-1000, XP002030812 ISSN: 0092-8674
- MORROW C S ET AL: "STRUCTURE OF THE HUMAN GENOMIC GLUTATHIONE S TRANSFERASE PI GENE" GENE (AMSTERDAM), vol. 75, no. 1, 1989, pages 3-12, XP001120420 ISSN: 0378-1119
- MILLAR D S ET AL: "DETAILED METHYLATION ANALYSIS OF THE GLUTATHIONE S-TRANSFERASE PI (GSTP1) GENE IN PROSTATE CANCER" ONCOGENE, BASINGSTOKE, HANTS, GB, vol. 18, no. 6, 11 February 1999 (1999-02-11), pages 1313-1324, XP000986829 ISSN: 0950-9232
- E.J. OAKELEY.: 'DNA methylation analysis: a review of current methodologies' PHARMACOLOGY AND THERAPEUTICS, vol. 84, 1999, pages 389 - 400, XP001029527
- N. OTA ET AL.: 'Determination of interactions between structured nucleic acids by fluorescence resonance energy transfer (FRET): selection of target sites for functional nucleic acids' NUCLEIC ACIDS RESEARCH, vol. 26, no. 3, 1998, pages 735 - 743, XP002131282
- S. TYAGI & F.R. KRAMER.: 'Molecular beacons: Probes that fluoresce upon hybridization' NATURE BIOTECHNOLOGY, vol. 14, 1996, pages 303 - 308, XP002918316

## Description

### Field of the Invention

The present invention relates to a method for detecting the presence of methylated nucleic acids. In particular, it relates to a method of diagnosing disease states in a patient, like cancer, where nucleic acids in diseased tissue become methylated.

### Background Art

### Scientific

Methylation of cytosine residues in DNA is currently thought to play an active role in controlling normal cellular development. Various studies have demonstrated that a close correlation exists between methylation and transcriptional inactivation. Considerable evidence exists to establish that in vertebrates, inactive genes often contain a modified cytosine residue 5-methylcytosine (mC) followed immediately by a guanosine (G) residue in the DNA sequence. Regions of DNA that are actively engaged in transcription, however, lack 5' methylcytosine residues.

There is now considerable evidence suggesting that alterations in the DNA methylating machinery in mammals may play an important role in tumourigenesis and tumour progression. In this respect, focal hypermethylation and generalised genomic demethylation are recognised features of many different types of neoplasms.

Targets for regional hypermethylation are normally unmethylated "CpG islands" located in gene promoter regions. This hypermethylation correlates with transcriptional repression that can serve as an alternative to coding region mutations for inactivation of tumor suppressor genes, including p16, p15, VHL, and E-cad. How general genomic hypomethylation and hypermethylation of some specific regions, in particular, evolve during tumourigenesis is just beginning to be defined. Normally, unmethylated CpG islands appear protected from dense methylation affecting immediate flanking regions. In neoplastic cells this protection is lost, possibly due to chronic exposure to increased DNA-methyltransferase activity and/or disruption of local protective mechanisms.

The hypermethylation of certain genes can also have a role to play in the control of the cell cycle. One such gene is the Myf-3 gene. The Myf-3 gene is normally hypomethylated in non-malignant tissues. Recent studies have shown that the Myf-3 gene is dramatically hypermethylated in many types of cancerous tissue. Therefore, a simple method of detecting hypermethylation of genes should provide new approaches to detection and diagnosis of some cancer.

Mapping of methylated regions in DNA has relied primarily on Southern Blotting techniques, based on the inability of methylation-sensitive restriction enzymes to cleave sequences that contain one or more methylated cytosine residues. This method is relatively insensitive, requires large amounts of high molecular weight DNA and can only provide information about those cytosine residues found within sequences recognized by methylation-sensitive restriction enzymes. A further disadvantage of the Southern Blotting methods is that the whole procedure requires 7-10 days.

The Polymerase Chain Reaction (PCR) has also been used to detect methylated DNA. In PCR methods, methylation sensitive enzymes are employed to distinguish between methylated and non-methylated DNA. More specifically, PCR primers are designed to span a region of DNA that includes a restriction endonuclease recognition sequence that is sensitive to DNA methylation. If the enzyme recognition sequence is not methylated the DNA is hydrolysed and the PCR target DNA is destroyed. If the DNA is methylated the enzyme does not hydrolyse the target and DNA chain synthesis is achieved. Restriction of unmethylated DNA must be complete, since any uncleaved DNA will be amplified by PCR. This can lead to a false positive result for methylation. A further problem is that this method involves multiple steps.

A third method combines PCR with bisulphite treatment of DNA to convert all unmethylated cytosines to thymine. Methylated cytosine residues are protected from conversion to thymine by bisulphite. PCR primers that are specific for converted or unconverted cytosine residues are used to generate DNA chain synthesis including the cytosine residues under investigation. Usually cloning and sequencing steps are required to assign which cytosine residues are methylated. This method is technically demanding, labour intensive and without cloning amplification products, requires approximately 10% of the alleles to be methylated for detection.

Current methods of detecting DNA methylation are time consuming, expensive and often tack specificity. The present invention seeks to ameliorate these and other problems associated with the prior art by providing a new an improved method for the detection of methylated nucleic acids.

### General

Those skilled in the art will appreciate that the invention described herein is susceptible to variations and modifications other than those specifically described.

The present invention is not to be limited in scope by the specific embodiments described herein, which are intended for the purpose of exemplification only. Functionally equivalent products, compositions and methods are clearly within the scope of the invention as described herein.

Bibliographic details of the publications numerically referred to in this specification are collected at the end of the description. No admission is made that any of the references constitute prior art.

As used herein the term "derived from" shall be taken to indicate that a specific integer may be obtained from a particular source *albeit* not necessarily directly from that source.

Throughout this specification and the claims that follow, unless the context requires otherwise, the word "comprise", or variations such as "comprises" or "comprising", will be understood to imply the inclusion of a stated integer or group of integers but not the exclusion of any other integer or group of integers.

### Summary of the Invention

The present invention provides a method for detecting methylated nucleic acids comprising the steps of:
1) Contacting a nucleic acid sample suspected of containing methylated nucleotides with an oligonucleotide sequence under suitable conditions for nucleic acid hybridization, said oligonucleotide sequence characterised in that,
   (i) it comprises a first stem labeled with a fluorophore moiety, a loop sequence having a region of nucleotides complementary to at least a region of the nucleic acid. sample, which region is susceptible to methylation, and a second stem labeled with a quencher moiety that is capable of quenching the fluorophore moiety when in spatial proximity to the fluorophore moiety; and
   (ii) the nucleotides forming the first stem are capable of moving into spatial proximity with the nucleotides forming the second stem when the probe is dissociated from the nucleic acid sample;
2) altering the hybridization conditions such that the oligonucleotide probe dissociates from unmethylated DNA but remains hybridized to methylated DNA; and
3) measuring the change in fluorescence.

### Brief Description of the Figures

In the drawings:

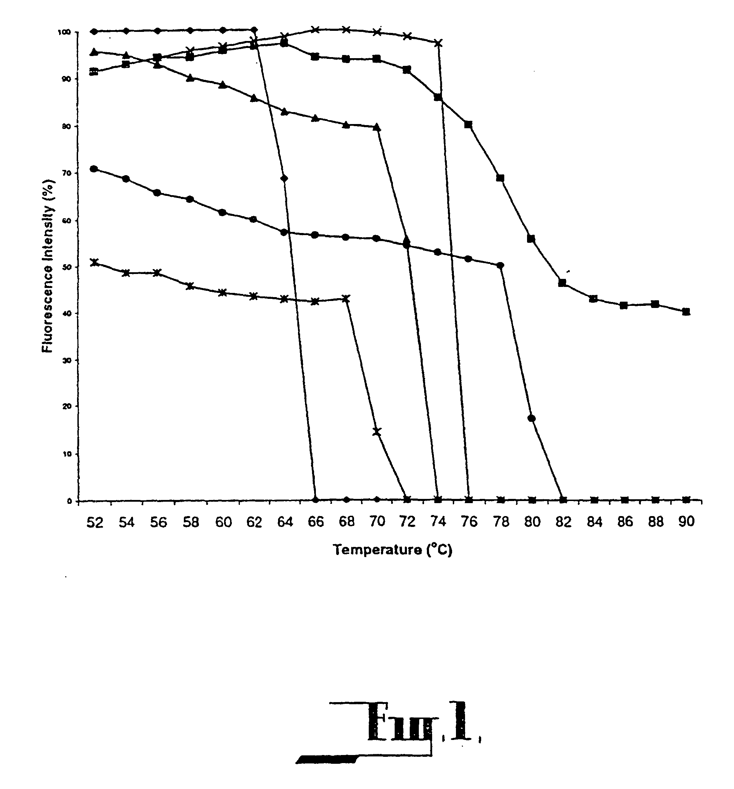

Figure 1 shows a denaturation profile of various reaction mixtures containing methylated and non-methylated target DNA sequences. As illustrated in the figure (◆) represents that use of molecular beacon B against an excess of target non-methylated Myf-3, (■) represents the use of molecular beacon B against an excess of target methylated Myf-3, (▲) represents molecular beacon A against an excess of target non-methylated Myf-3, (×) represents the use of molecular beacon A against an excess of target methylated Myf-3, (∗) represents the use of an equal amount of target against non-methylated Myf-3, and (●) represents molecular beacon A against an equal amount of target methylated Myf-3.

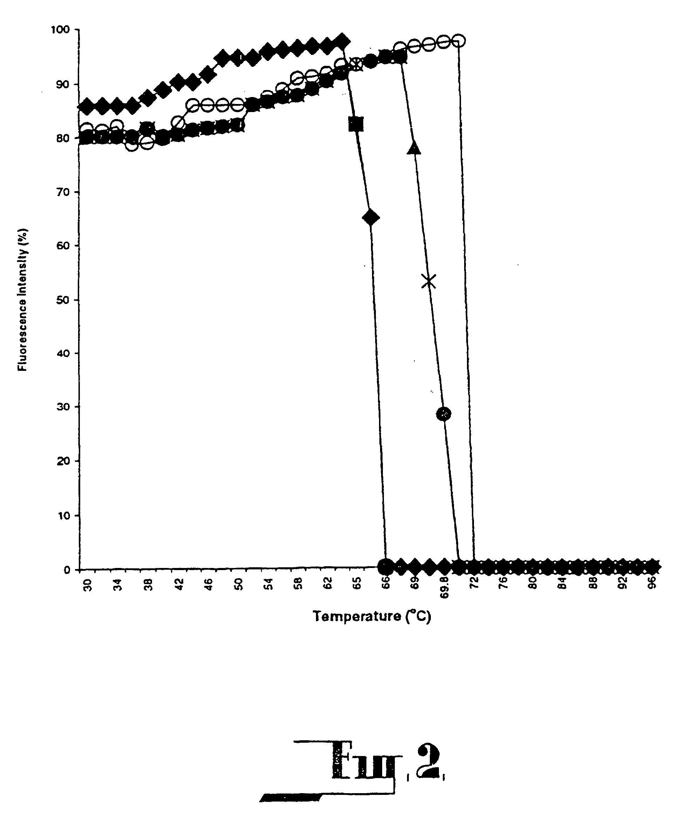

Figure 2 shows the denaturation profile of molecular beacon A with none, 1, 2, 3, 4 and 5, 5-methylated oligonucleotides. As illustrated in the figure (■) represents the use of molecular beacon A against the preferred Myf-3 target with no methylated cytosines, (◆) represents the use of molecular beacon A against the preferred Myf-3 target with one 5-methylated cytosine, (▲) represents the use of molecular beacon A against the preferred Myf-3 target with two 5-methylated cytosines, (•) represents the use of molecular beacon A against the preferred Myf-3 target with three methylated cytosines, (×) represents the use of molecular beacon A against the preferred Myf-3 target with four methylated cytosines, and (○) represents the use of molecular beacon A against the preferred Myf-3 target with five methylated cytosines.

### Detailed Description of the Invention

According to the present invention there is provided a method for identifying nucleic acid sequences that have been methylated. Notably this technology has been found to be sensitive enough to detect and or differentiate between methylated and non-methylated nucleic acid sequences where methylation has occurred with as few as a single cytosine residue.

Such technology may have wide ranging application as a means to detect the presence of methylated nucleic acid sequences in an oligonucleotide sample. Methylation of nucleic acid sequences has been found to occur, for example, in cancerous tissue and when foreign DNA is introduced into a host, especially by way of viral infection. In fact, it is now recognised that DNA methylation is one of the ways in which introduced foreign DNA, especially by way of a virus, is neutralised or silenced. Where either a cells DNA (eg in cancer) or foreign is methylated, the present invention may be used to detect the presence of the methylated DNA.

Since DNA methylation influences gene expression, therapeutic strategies aimed at reactivating or suppressing specific genes associated with particular disease states will most likely require access to a simple method for measuring DNA methylation, the present invention provides such a method. In addition, studies aimed at understanding the most appropriate ways of promoting gene therapy may also be advanced by the availability of a simple method for detection of methylated cytosine residues, such as that described herein.

Methylation of cytosine also plays an important role in normalisation of gene dosage. For example, in females, one of the X-chromosomes is maintained in an inactive form by cytosine methylation. DNA methylation is one of the major gene silencing mechanisms involved in parental imprinting. In order to maintain the appropriate expression of genes such as H19 for example, one of the maternally or paternally inherited genes are silenced by DNA methylation. DNA methylation also plays a vital role in many biological processes during development. For example, experimental reduction in the ability to methylate cytosine residues within DNA results in embryological death.

Thus, the present invention provides a method for detecting methylated nucleic acids comprising the steps of:
1) contacting a nucleic acid sample suspected of containing methylated nucleotides with an oligonucleotide sequence under suitable conditions for nucleic acid hybridization, said oligonucleotide sequence characterised in that,
   (i) it comprises a first stem labeled with a fluorophore moiety, a loop sequence having a region of nucleotides complementary. to at least a region of the nucleic acid sample, which region is susceptible to methylation, and a second stem labeled with a quencher moiety that is capable of quenching the fluorophore moiety when in spatial proximity to the fluorophore moiety; and
   (ii) the nucleotides forming the first stem are capable of moving into spatial proximity with the nucleotides forming the second stem when the probe is dissociated from the nucleic acid sample;
2) altering the hybridization conditions such that the oligonucleotide probe dissociates from unmethylated DNA but remains hybridized to methylated DNA; and
3) measuring the change in fluorescence.

Preferably, when labeled oligonucleotide sequences dissociate from the target nucleic acid sample the first and second stem hybridise together causing quenching of the fluorophore moiety.

When the loop sequence in the probe binds a complementary sequence in a target gene the probe enters an "open conformation" and fluorescence of the donor fluorophore is detectable. When the probe is in a closed (hairpin) conformation, the fluorescence of the donor fluorophore is quenched.

Desirably the loop sequence is complementary to a portion of a nucleic acid sequence that undergoes methylation when a cell transforms from a normal state to a cancerous state. Further the loop sequence is preferably selected such that it is capable of specifically hybridizing with the target sequence, but is unable to form internal structures that favour maintenance of the probe in a "closed configuration" (ie when the two stems hybridise together). In a particularly preferred probe design, the probe is designed to hybridise to a region in a nucleic acid sequence that has a high proportion of CG oligonucleotides that have the potential to be methylated.

In one preferred embodiment, the present invention may be used to detect the presence cancerous cells in a tissue sample. Methylation of various genes has been implicated in the onset or development of cancerous states in various cells. One such gene that is known to undergo such a transformation is the Myf-3 gene. Other genes that may contain regions of DNA that are normally unmethylated but become hypermethylated in neoplastic cells include, for example, the PAX genes, calcitonin and glutathione-S-transferase-Π(pi) genes.

Specific cytosine residues within a particular region of glutathione-S-transferase (pi) have recently been shown to be exclusively methylated in malignant prostate tissue (1). Identification of the cytosine residues within glutathione-S-transferase (pi) that are abnormally methylated in prostate cancer tissues provides a new method for the diagnosis of malignancy of prostate tissue.

As a gene becomes methylated there is a progressive increase in the melting temperature of the gene and its complement. The present invention capitalises on this characteristic to distinguish unmethylated and methylated nucleic acids.

When a labeled oligonucleotide sequence, as described herein, encounters a target complementary DNA sequence, it should desirably form a hybrid with that sequence that is stronger and more stable than the hybrid formed by stem sequences. When this happens the labeled oligonucleotide sequence undergoes a spontaneous conformational change that results in the stem sequences moving away from each other, causing the labeled oligonucleotide sequence to enter an "open conformation," wherein fluorescence can be detected since the fluorophore is no longer in close proximity to the quencher. According to the present invention unhybridized labeled oligonucleotide sequence should not be capable of fluoresceing.

Detection of methylation according to the invention is achieved by altering hybridization conditions during a hybridization reaction to facilitate dissociation of labeled oligonucleotide sequence from an oligonucleotide sample. Preferably achieved by raising the temperature of the hybridization reaction. As the temperature increases, a temperature will be reached where labeled oligonucleotide sequence melts away from an unmethylated target sequence but remains bound to methylated DNA.

Dissociation of labeled oligonucleotide sequence from the target provides the labeled oligonucleotide sequence with conformational freedom permitting it to form a closed conformational state, which results in a reduction in fluorescence. Specifically, alteration in the structure of the labeled oligonucleotide sequence brings the fluorophore and the quencher into a spatial proximity that quenches fluorescence. By measuring a change in fluorescence in the reaction as the temperature of the hybridization reaction is increased, it is possible to detect when a probe melts away from its complementary sequence. Since methylated DNA dissociates at a higher temperature it is possible to distinguish methylated from unmethylated DNA.

The melting temperature of the labeled oligonucleotide sequence will depend upon the length and the G-C content of the loop and stem sequences and the concentration of the salts in the solution in which it is dissolved.

Where the melting temperature of a particular labeled oligonucleotide sequence when annealed to a methylated and an unmethylated gene is known, the hybridization conditions may be varied by increasing the hybridization temperature to the temperature that causes the loop sequence to melt away from unmethylated complementary target gene sequence but not methylated target gene sequence. Measurements should then be taken of the amount of fluorescence in the reaction mixture and the results should be analysed. Because the stem sequences are adapted to come together following separation from unmethylated gene sequence a drop in fluorescence should be observed.

Where the melting temperature of a labeled oligonucleotide sequence bound to a methylated and/or unmethylated nucleic acid sequence is not known or there is uncertainty about the melting temperature, a plurality of different control reactions are preferably performed in parallel with the test sample. The control reactions should contain at least a corresponding gene sequence that is either unmethylated or methylated or, if desired, both unmethylated and methylated reactions may be run as two separate controls.

The term "molecular beacon" as used herein refers to a molecule capable of participating in a specific binding reaction and whose fluorescent activity changes when the molecule participates in that binding reaction. The labeled oligonucleotide sequence referred to above is for the purposes of this invention a molecular beacon.

A labeled oligonucleotide sequence is "hybridizable" to a nucleic acid molecule, such as a cDNA, genomic DNA, or RNA, when a single-stranded form of the sequence can anneal to the nucleic acid molecule under the appropriate conditions of temperature and solution ionic strength (see Sambrook *et al*., 1989, (2)). The conditions of temperature and ionic strength determine the "stringency" of the hybridization. An example of progressively higher stringency conditions is as follows: 2 x SSC/0.1% SDS at about room temperature (hybridization conditions); 0.2 x SSC/0.1% SDS at about room temperature (low stringency conditions); 0.2 x SSC/0.1% SDS at about 42°C (moderate stringency conditions); and 0.1 x SSC at about 68°C (high stringency conditions). Hybridization requires that the probe and the nucleic acid molecule contain complementary sequences, although depending on the stringency of the hybridization, mismatches between bases are possible. The appropriate stringency for hybridizing nucleic acids depends on variables well known in the art. For example, the length, degree of complementarity, nucleotide sequence composition (e.g., GC v. AT content), and nucleic acid type (e.g., RNA v. DNA) of the hybridizing regions of the nucleic acids can be considered in selecting hybridization conditions. The greater the degree of complementary between two nucleotide sequences, the greater the value of Tₘ for hybrids of nucleic acids having those sequences. The relative stability (corresponding to higher Tₘ) of nucleic acid hybridizations decreases in the following order: RNA:RNA, DNA:RNA, DNA:DNA. For hybrids of greater than 100 nucleotides in length, equations for calculating Tₘ have been derived (*see* Sambrook et al., 1989, *supra,* 9.50-9.51). For hybridization with shorter nucleic acids, the position of mismatches becomes more important, and the length of the oligonucleotide determines its specificity (see Sambrook et al., 1989, *supra,* 11.7-11.8). Preferably the loop sequence comprises at least a minimum number of nucleotides to avoid non-specific binding between the probe and its target nucleic acid sequence. Desirably a minimum length for a hybridizable nucleic acid is at least about 10 nucleotides; more preferably at least about 15 nucleotides; most preferably the length is at least about 20 nucleotides. In a highly preferred form of the invention the loop sequence is a structure of approximately 18 or 19 bases.

While the loop sequence should contain at least about 10 nucleotides, the stem sequence, may comprise any nucleic acid sequence that ensures hybridization between the two stems when in a free (ie. unbound) state. In this respect, the stems need not be of equal length. If the stems are of different lengths, they must be capable of bringing the fluorophore and the quencher into spatial proximity to quench the fluorescence of the fluorophore when the two stems hybridize to each other. In one form of the invention the stem sequences will not hybridise to the probe's target gene and are of a sufficiently short length to avoid non-specific binding between the stem and any other nucleic acid sequence in the reaction mixture. In another form of the invention one of the stems may be adapted to hybridise to the target gene sequence.

The minimum and maximum lengths of the stems should preferably be about 4 and 8 nucleotides respectively. Further, the stem sequence may contain one or more methylated cytosine residues that are capable of aiding the stem sequences to hybridize when brought into spatial proximity of each other.

Mg⁺⁺ has a powerful stabilizing influence on the stem hybrid. In a particularly preferred embodiment of the invention the hybridization reaction is carried out in the presence of a sufficient concentration of Mg⁺⁺ ions that is capable of facilitating stem hybrid formation. It is preferable that the reaction mixture contains MgCl₂ to increase the stability of the hybrid. Preferably the concentration of MgCl₂ is 5mM to 10mM.

A further consideration for the length of the probe as a whole will be the need to create a minimum distance between the fluorophore and the quencher moieties used in the probe when in the open conformation to avoid quenching of the fluorophore. This optimal distance will vary with the specific moieties used, and may be easily determined by one of ordinary skill in the art using techniques known in the art. Desirably, the loop sequence is at least twice the length of the stem sequences to ensure that the conformational change occurs upon hybridization and to ensure that the fluorophore is sufficiently far from the quencher to restore full fluorescence. Preferably the moieties are separated by a distance of up to 35 nucleotides, more preferably from 10-25nucleotides, and still more preferably from 15-20 nucleotides.

To avoid false positives in the method, the amount of probe employed in the reaction is desirably at least equal to or greater than the relative amount of target nucleotide sequence in the sample. Determining the concentration of target nucteotide sequence in the sample may be achieved by any method known in the art.

Oligonucleotide probes used in the invention can be synthesized by a number of approaches known in the art (see, for example 3,4). The oligonucleotide probes of the invention are conveniently synthesized on an automated DNA synthesizer, e.g. an Applied Biosystems, Inc. Foster City, Calif.) model 392 or 394 DNA/RNA Synthesizer, using standard chemistries, such as phosphoramidite chemistry, e.g. disclosed in the following references: Beaucage and lyer, (1992) Tetrahedron, 48:2223-2311; U.S. Pat. No. 4,980,460; U.S. Pat. No. 4,725, 677; U.S. Pat. Nos. 4,415,732; 4,458,066 and 4,973,679; and the like. Alternative chemistries, e.g. resulting in non-natural backbone groups, such as phosphorothioate, phosphoramidate, and the like, may also be employed provided that the hybridization efficiencies of the resulting oligonucleotides and/or cleavage efficiency of the exonuclease employed are not adversely affected.

The fluorophore and the quencher may be attached to the probe by any means that enables quenching of the fluorophore when in a closed conformation and illumination of the fluorophore in an open conformation. For example the moieties may be attached to the probe by chemical linkers etc. Preferably the fluorophore and or the quencher molecule are attached to the 5' or 3' terminal nucleotide in the probe. Methods for attaching such moieties to a nucleotide probe are known to those skilled in the field.

A fluorophore is a chemical compound which when excited by exposure to particular wavelengths of light, emits light (ie. fluoresces) at a different wavelength. Fluorophores and quencher molecules participate in fluorescence resonance energy transfer (FRET).

In FRET, energy is passed non-radioactively over a long distance (1 - 10nm) between the flurophore and a quencher molecule. The fluorophore absorbs a photon and transfers this energy non-radioactively to the quencher. When the fluorophore and the quencher are in close proximity and the emission and absorption spectra overlap the energy of the fluorophore is transferred to the quencher without subsequent emission of fluorescence. Preferably, the nature of the fluorophore-quencher pair is such that energy received by the fluorophore is transferred to the quencher and dissipated as heat, rather than being emitted as light. As a result the fluorophore is unable to fluoresce.

Combinations of a fluorophore and an interacting molecule such as a quenching moiety are known as "FRET" pairs. The primary requirement for FRET is that the emission spectrum of the fluorophore overlaps with the absorption spectrum of the quenching molecule. The efficiency of energy transfer decreases proportionately to the sixth power of the distance between the fluorophore and quencher molecules. One of ordinary skill in the art can easily determine, using art-known techniques of spectrophotometry, which fluorophores and which quenchers will make suitable FRET pairs. Molecules that are commonly used in FRET pairs include fluorescein, 5-carboxyfluorescein (FAM), 2'7'-dimethoxy-4'5'dichloro-6-carboxyfluoroscein (JOE), rhodamine, 6'carboxyrhodamine (R6G), N,N,N',N'-tetramthyl -6-carboxyrhodamine (TAMRA), 6-carboxy-X-rhodamine (ROX), 4-(4'-dimethylaminophenylazo) benzoic acid (DABCYL), and 5-(2'-aminoethyl)aminonaphthalene-1-suffonic acid (EDANS). Preferably, FAM (which has an emission maximum of 525 nm) is used as a fluorophore for TAMRA, ROX, DABCYL and R6G (all of which have an excitation maximum of 514 nm) in a FRET pair. Alternative the fluorophore may be EDANS and the quencher may be DABCYL.

The above method has general application to detect any DNA sequence which is methylated in cancerous tissue while hypomethylated in non-cancerous tissue. In one embodiment of the invention the labeled oligonucleotide sequence is directed against at least a target sequence in the Myf-3 gene. Preferable target sequences of the Myf-3 gene are as follows:

5' GCG GCG ACT CCG ACG CGT CCA GCC CGC GCT CC 3'

5' TTA TAC CGC AGG CGG GCG AGC CGC GGG CGC TCG CT 3'

5' CCG AGA GCC CTG CGG GGC CCG CCC TCC TGC TGG CG 3'

In another embodiment of the invention the labeled oligonucleotide sequence is directed against at least a target sequence in the glutathione-S-transferase (pi) gene. Preferable target sequences of the glutathione-S-transferase (pi) gene would be as follows:

5' CTC CAG CGA AGG CCT CGC GGC CTC CGA GCC TTA TAA G 3'

5' GGG GAC GCG GGC CGC GCG TAC TCA CTG GTG GCG A 3'

The above sequences are particularly preferred targets for molecular beacons for the following reasons:
i) they contain a high density of CG dinucleotides which have the potential to be methylated;
ii) the intervening sequences include sufficient numbers of the nucleotides A and T in order to make the beacon specific for Myf-3 or glutathione-S-transferase(pi)
iii) beacons designed to hybridise with these regions are mostly unable to form any inappropriate internal structures that may favour maintenance of the beacon in a closed configuration

In a highly preferred form of the invention the temperature of the hybridization reaction is maintained at 20°C for 20 minutes and increased from 25°C to 45°C at increments of 2° with a 2 minute stabilising time. As the temperature is increased from 50°C to 90°C at increments of 2°C every 5 minutes the fluorescence of the reaction mix is monitored. Still preferably, the temperature is allowed to increase from 50°C to 95°C in 2' increments with each temperature being held for 1 minute.

### Best Method of Performing the Invention

Further features of the present invention will be more fully described in the following Examples. It is to be understood, however, that this detailed description is included solely for the purposes of exemplifying the invention, and should not be understood in any way as a restriction on the broad description as set out above. In particular, it will be understood that all temperature ranges and other such variables prescribed in the examples are given as indicative only, and that parameters outside these limits may also provide useful results.

Molecular biological methods that are not explicitly described in the following Examples are reported in the literature and are known by those skilled in the art. General texts that described conventional molecular biology, microbiology, and recombinant DNA techniques within the skill of the art, included, for example: Sambrobk *et al*., *Molecular Cloning: A Laboratory Manual,* Second Edition, Cold Spring Harbor Laboratory Press, Cold Spring Harbor, New York (1989); Glover ed., *DNA Cloning: A Practical Approach,* Volumes I and II, MRL Press, Ltd., Oxford, U.K. (1985); and Ausubel, F., Brent, R., Kingston, R.E., Moore, D.D., Seidman, J.G., Smith, J.A., Struhl, K. *Current protocols in molecular biology.* Greene Publishing Associates/Wiley Intersciences, New York.

### EXAMPLES

### Probes

Two molecular beacons with loop sequences complementary to the preferred region of the Myf-3 gene were purchased from the Midland Certified reagent company. Molecular beacon A contained a 18-nucleotide long complementary loop sequence and a 6 nucleotide-stem sequence (see Table 1). Five of the 12 stem nucleotides were also complementary to the target sequence.

Molecular beacon B contained the same 18-nucleotide complementary loop sequence with a 5-nucleotide stem sequence (see Table 1). The probe sequence is one base longer in molecular beacon A than in B with the extra base creating an extra base pair adjacent to the stem. Four of the 10 stem nucleotides were also complementary to the target sequence.

### Target DNA

To investigate the temperature profiles of methylated and non methylated DNA using molecular beacons, oligonucleotides of 32 bases representing the preferred region of the Myf-3 gene and complementary to the molecular beacons were purchased from Bresatec. The oligonucleotides contain 7 CG sites which include cytosine residues that are able to be methylated. In the 5M oligonucleotide, cytosines within each of the 7CG dinucleotides were replaced with 5-methylated cytosines. The Non-5M oligo remained unmethylated. All the oligonucleotides and the molecular beacons purchased were HPLC purified.

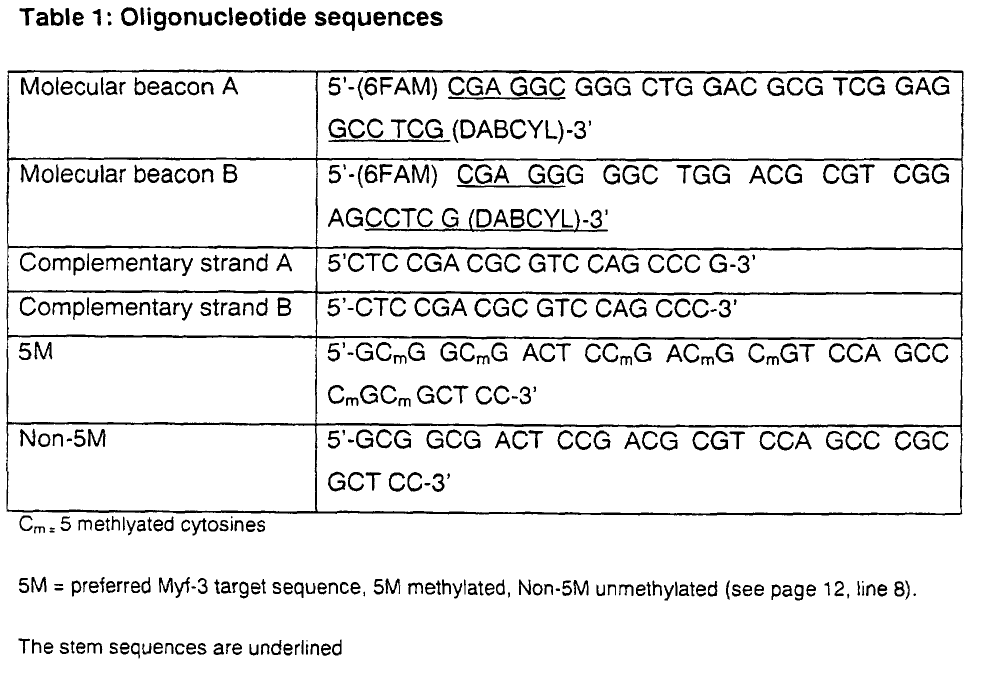

### Thermal denaturation profiles

Molecular beacons are known to linearize and hybridize with complementary DNA at certain temperatures releasing fluorescence as an indicator of hybridization. In order to determine the effects of changing temperatures on the level of fluorescence, thermal denaturation profiles were carried out with different oligonucleotides and molecular beacons. Three different temperature conditions were investigated and the best denaturation profile was chosen for further study. The fluorescence was monitored during each profile by using a fluorescence reader with a programmed temperature control (PE/ABI 7700 Applied Biosystems).

The first profile included increasing the temperature from 55°C-95°C at 1° steps with each temperature being held for 1 minute. In the second profile, the temperature was maintained at 20°C for 20 minutes and increased from 25°C to 45°C at increments of 2° with a 2 minute stabilising time. The fluorescence was monitored as the temperature was increased from 50°C to 90°C at increments of 2° every 5 minutes. In another profile the same steps were carried out with the fluorescence detected as the temperature was increased at increments of 1° every 5 minutes.

The fluorescence intensity of at each temperature was plotted as a function of temperature on a linear scale from 0-100%. Representative profiles are shown in Fig. 1.

### Reaction mix

Melting curve experiments were conducted by adding appropriate concentrations of oligonucleotides to a reaction mixture containing 20mM tris-HCl (pH 8.0), 50mM KCl and 5mM MgCl₂. The molecular beacons were added to a final concentration of 3µM. The reaction mixtures were made to a final volume of 50 µl with double distilled water. The beacon was added to the reaction mixture at the very last minute, in order to minimise any molecular beacon/target interaction prior to the temperature control.

### Hybridization Conditions

In order to determine the hybridization efficiency and the minimum amount of oligonucleotide needed for hybridization, the oligonucleotides were added in equal, limited and excess amounts to the reaction mixture (ie. Equal: 3µM, Limited: 2µM, Excess: 6µM, 12µM respectively).

### Determination of the effects of salt concentration

Divalent cations such as Mg⁺⁺ are known to have powerful stabilising effect on the stem hybrids. The effects of different salt concentrations on denaturation profiles of molecular beacon B with strand A and B were investigated. Reaction mixtures containing equal, limited and excess amounts of oligonucleotides were subjected to 10mM, 5mM and 0mM concentrations of MgCl₂.

### Thermal denaturation profiles with methylated oligonucleotides

Molecular beacon A and B were added to a final concentration of 3µM to reaction mixtures containing equal, limited and excess amounts of methylated oligonucleotide, (5M) and non-methylated oligonucleotides, (non-5M) (3µM, 1.4µM and 6µM respectively). The reaction mixtures were then subjected to the temperature profile: 20°C for 20min, 25°C-35°C in 2° increments each 2min, and 50°C to 90°C at 2° increments every 5min where fluorescence was monitored.

### Determination of the Hybridisation Profile

In order to construct a denaturation profile for the molecular beacons with complementary strand A and B, the temperature was allowed to increase from 50°C to 95°C in 2° increments with each temperature being held for 1min. This resulted in a typical sigmoid curve. When the temperature was raised, the fluorescence was increased. This was due to a conformational change of the molecular beacon from the stem loop structure to a random coil structure.

According to Tyagi et al., (5) molecular beacons hybridise spontaneously to their targets at room temperature. Therefore the mixture was incubated initially at 20°C for 20 min to allow for effective hybridisation of the target to the molecular beacon. The fluorescence reaches a maximum at approximately 50°C.

It remained high until a certain temperature was reached, where the % fluorescence decreased rapidly or reached 0% fluorescence. This temperature represented the temperature at which the probe-target hybrid melts. For molecular beacon B with complementary strand A and B the hybridisation melt temperature was approximately 74°C-76°C. The hybridisation melting temperature for molecular beacon B was approximately 74°C. In the above incidences the % fluorescence reached 0% after the hybridisation melt temperature was reached. On the other hand % fluorescence of beacon A with strand A dropped around 76°C-78°C but did not reach 0% even at 90°C.

With the above mentioned temperature profile it was possible to measure consistent temperature profiles as well as allowing enough time for molecular beacon-target hybridisation.

### The amounts of oligonucleotide needed for hybridisation

In order to determine the minimum amounts of oligonucleotide necessary for hybridisation, molecular beacons A and B were added to reaction mixtures containing equal, limited and excess amounts of oligonucleotide. No distinct hybridisation melt temperature was observed in limited (1.5µM or 0.8µM) oligonucleotide samples with the exception of 1.5µM oligonucleotide sample with molecular beacon B. A hybridisation melt temperature of approximately 66°C with complementary strand A and approximately 72°C with complementary strand B was observed.

The maximum fluorescence intensity reached with equal amounts of oligonucleotides was lower than the reaction mixtures with excess oligonucleotide. The hybridisation temperature was also elevated in the excess oligonucleotide samples. For example molecular beacon with equal amounts of strand A had a hybridisation temperature of 66°C, while for the same reaction mixture with excess strand had a melt temperature of approximately 74°C. It was generally concluded that for effective molecular beacon-target hybridisation to occur, it is necessary to have the target either in excess or at least in equal amounts.

### Effects of salt concentrations on the denaturation profiles

Analysis of different salt concentrations with molecular beacon B with strand A and B indicated that the presence of MgCl₂ in the reaction mixture increases the stability of the hybrids as predicted. In both limited and equal amounts of oligonucleotide, a higher intensity of fluorescence was detected in samples with 5mM MgCl₂ concentration. But when the salt concentration was increased from 5mM to 10mM only a marginal increase in fluorescence intensity was observed.

### Methylation detection

The hybridisation melting temperature observed when molecular beacons A and B were hybridised to the methylated oligonucleotide 5M was higher than the melting temperatures of beacons A and B hybridised to the non-methylated oligonucleotide, Non-5M.

For example, as shown in Fig. 1, molecular beacon A with an equal amount of 5M melted at approximately 80°C while the non-methylated Non-5M melted at approximately 69°C. Molecular beacon A with excess amounts of 5M had a hybridisation melt temperature of approximately 76°C while for the non-5M it was approximately 71°C. With molecular beacon B and excess amounts of 5M, the hybridisation started to melt at approximately 68°C with the fluorescence intensity decreasing at a slower rate. With excess non5-M the melting temperature was approximately 64°C.

### Sensitivity of Detection of Methylated Cytosines

The molecular beacon method can readily detect methylation of the preferred target DNA when all 7 cytosine resides within the CpG dinucleotides are methylated (see Table 1). To determine the minimum number of cytosine residues that need to be methylated in order to alter the melt temperature of hybrids, preferred Myf-3 targets with variable numbers of methylated cytosine residues were reacted with beacon A in the test system. As shown in Table 2, five different modified preferred Myf-3 targets were examined. Modifications ranged from having one to five cytosine residues methylated. As illustrated in Fig. 2, beacon A hybrids with targets that have 2 or more cytosine residues dissociate at a significantly higher melt temperature compared to targets which have 1 or no methylated cytosine residues.

### Methylated Oligonucleotides

                                    M     M   M                 M   M
5-5M        5' - GCG GCG ACT CCG ACG CGT CCA GCC CGC GCT CC - 3'

M M M M
4-5M 5' - GCG GCG ACT CCG ACG CGT CCA GCC CGC GCT CC - 3'

M M M
3-5M 5' - GCG GCG ACT CCG ACG CGT CCA GCC CGC GCT CC - 3'

M M
2-5M 5' - GCG GCG ACT CCG ACG CGT CCA GCC CGC GCT CC - 3'

M
1-5M 5' - GCG GCG ACT CCG ACG CGT CCA GCC CGC GCT CC - 3'

M=methylated cytosine residues.
NB. Reaction profiles shown in Fig. 2.

### References

1. Millar *et al*, (1999) Oncogene, 18:1313-1324
2. Sambrook *et al*., *Molecular Cloning: A Laboratory Manual,* Second Edition, Cold Spring Harbor Laboratory Press, Cold Spring Harbor, New York (1989)
3. Ozaki *et al.* (1992) Nucleic Acids Research, 20:5205-5214
4. Agrawal *et al.* (1990) Nucleic Acids Research, 15:5319-5423
5. Tyagi et al., *Nature* 1996 14(3): 303-308

## Claims

1. A method for detecting methylated nucleic acids comprising the steps of:
(i) contacting a nucleic acid sample suspected of containing Methylated nucleotides with an oligonucleotide sequence under suitable conditions for nucleic acid hybridization, said oligonucleotide sequence **characterised in that**,
(a) it comprises a first stem labeled with a fluorophore moiety, a loop sequence having a region of nucleotides complementary to at least a region of the nucleic acid sample, which region is susceptible to methylation, and a second stem labeled with a quencher moiety that is capable of quenching the fluorophore moiety when in spatial proximity to the fluorophore moiety; and
(b) the nucleotides forming the first stem are capable of moving into spatial proximity with the nucleotides forming the second stem when the probe is dissociated from the nucleic acid sample;
(ii) altering the hybridization conditions such that the oligonucleotide probe dissociates from unmethylated DNA but remains hybridized to methylated DNA; and
(iii) measuring the change in fluorescence

2. A method according to Claim 1, in which when the labeled oligonucleotide sequences dissociate from the target nucleic acid sample according to step (ii) the first and second stem hybridise together causing quenching of the fluorophore moiety.

3. A method according to Claim 1 or 2, in which the loop sequence contains at least about 10 nucleotides.

4. A method according to Claim 1 or 2, in which the loop sequence contains up to 35 nucleotides.

5. A method according to Claim 1 or 2, in which the loop sequence contains between 10 and 25 nucleotides.

6. A method according to claim 1 wherein the loop sequence contains at least about from 15-20 nucleotides.

7. A method according to claim 1 wherein when the loop sequence is complementary to a portion of a nucleic acid sequence that undergoes methylation when a cell transforms from a normal state to a cancerous state.

8. A method according to claim 1 wherein when the loop sequence is complementary to a portion of a Myf-3 nucleic acid sequence that undergoes methylation when a cell transforms from a normal state to a cancerous state.

9. A method according to claim 8 wherein the labelled oligonucleotide sequence is complementary to at least one of the sequences selected from the group consisting of:
(i) 5' GCG GCG ACT CCG ACG CGT CCA GCC CGC GCT CC 3'
(ii) 5' TTA TAC CGC AGG CGG GCG AGC CGC GGG CGC TCG CT 3'
(iii)

5' CCG AGA GCC CTG CGG GGC CCG CCC TCC TGC TGG CG 3'

10. A method according to claim 1 wherein when the loop sequence is complementary to a portion of a glutathione-S-transferase-Π(pi) nucleic acid sequence that undergoes methylation when a cell transforms from a normal state to a cancerous state.

11. A method according to claim 10 wherein the labelled oligonucleotide sequence is complementary to at least one of the sequences selected from the group consisting of:
i) 5' CTC CAG CGA AGG CCT CGC GGC CTC CGA GCC TTA TAA G 3'
ii)

5’ GGG GAC GCG GGC CGC GCG TAC TCA CTG GTG GCG A 3’

12. A method according to claim 1 wherein when the loop sequence is complementary to a portion of a calcitonin nucleic acid sequence that undergoes methylation when a cell transforms from a normal state to a cancerous state.

13. A method according to claim 1 wherein the method is used to detect abnormally methylated gene sequences in prostate cancer tissues.

14. A method according to any preceding claim, in which the hybridization condition that is altered during the hybridization reaction is the temperature of the hybridization reaction.

15. A method according to any preceding claim, in which the stem sequences do not hybridise to the target gene and are of a sufficiently short length to avoid non-specific binding between the stem and any other nucleic acid sequence in the reaction mixture.

16. A method according to any preceding claim, in which the stem sequences are between about 4 to 8 nucleotides in length.

17. A method according to any preceding claim, in which at least a cytosine in at least one of the stem sequences is a methylated cytosine residue.

## Patentansprüche

1. Verfahren zur Detektion methylierter Nukleinsäuren, die Schritte umfassend:
(i) in Kontakt bringen einer Nukleinsäureprobe, von der man vermutet, dass sie methylierte Nukleotide enthält, mit einer Oligonukleotidsequenz unter Bedingungen, die zur Nukleinsäurehybridisierung geeignet sind, wobei diese Oligonukleotidsequenz **dadurch gekennzeichnet ist, dass**
(a) sie einen ersten Stamm, der mit einem Fluorophor-Anteil markiert ist, eine Loop-Sequenz, die eine Region von Nukleotiden besitzt, welche komplementär zu mindestens einer Region der Nukleinsäureprobe ist, wobei diese Region der Methylierung zugänglich ist, und einen zweiten Stamm umfasst, der mit einem Quencher-Anteil markiert ist, welcher in der Lage ist den Fluorophor-Anteil zu löschen, wenn er sich in räumlicher Nähe zum Fluorophor-Anteil befindet, und
(b) die Nukleotide, welche den ersten Stamm bilden, in der Lage sind sich in räumliche Nähe zu den Nukleotiden zu bewegen, die den zweiten Stamm bilden, wenn die Sonde von der Nukleinsäureprobe abgetrennt wird;
(ii) Ändern der Hybridisierungsbedingungen, so dass die Oligonukleotid-Sonde von der unmethylierten DNA dissoziiert aber an die methylierte DNA hybridisiert bleibt, und
(iii) Messen der Änderung der Fluoreszenz.

2. Verfahren gemäß Anspruch 1, in welchem die markierten Oligonukleotidsequenzen von der Targetnukleinsäureprobe gemäß Schritt (ii) dissoziieren und der erste und zweite Stamm miteinander hybridisieren, wodurch die Löschung des Fluorophor-Anteils bewirkt wird.

3. Verfahren gemäß Anspruch 1 oder 2, in welchem die Loop-Sequenz wenigstens ungefähr 10 Nukleotide enthält.

4. Verfahren gemäß Anspruch 1 oder 2, in welchem die Loop-Sequenz bis zu 35 Nukleotide enthält.

5. Verfahren gemäß Anspruch 1 oder 2, in welchem die Loop-Sequenz zwischen 10 und 25 Nukleotide enthält.

6. Verfahren gemäß Anspruch 1, worin die Loop-Sequenz wenigstens ungefähr 15-20 Nukleotide enthält.

7. Verfahren gemäß Anspruch 1, worin die Loop-Sequenz komplementär zu einem Teil einer Nukleinsäuresequenz ist, welche der Methylierung unterliegt, wenn eine Zelle von einem normalen Zustand in einen kanzerösen Zustand übergeht.

8. Verfahren gemäß Anspruch 1, worin die Loop-Sequenz komplementär zu einem Teil einer Myf-3-Nukleinsäuresequenz ist, welche der Methylierung unterliegt, wenn eine Zelle von einem normalen Zustand in einen kanzerösen Zustand übergeht.

9. Verfahren gemäß Anspruch 8, worin die markierte Oligonukleotidsequenz komplementär zu wenigstens einer der Sequenzen ist, ausgewählt aus der Gruppe, bestehend aus:
(i) 5' GCG GCG ACT CCG ACG CGT CCA GCC CGC GCT CC 3'
(ii) 
5' TTA TAC CGC AGG CGG GCG AGC CGC GGG CGC TCG
CT 3'
(iii)

5' CCG AGA GCC CTG CGG GGC CCG CCC TCC TGC TGG
CG 3'

10. Verfahren gemäß Anspruch 1, worin die Loop-Sequenz komplementär zu einem Teil einer Glutathion-S-Transferase-Π- (pi) -Nukleinsäuresequenz ist, welche der Methylierung unterliegt, wenn eine Zelle von einem normalen Zustand in einen kanzerösen Zustand übergeht.

11. Verfahren gemäß Anspruch 10, worin die markierte Oligonukleotidsequenz komplementär zu wenigstens einer der Sequenzen ist, ausgewählt aus der Gruppe bestehend aus:
i) 
5' CTC CAG CGA AGG CCT CGC GGC CTC CGA GCC TTA
TAA G 3'
ii)

5' GGG GAC GCG GGC CGC GCG TAC TCA CTG GTG GCG A
3'

12. Verfahren gemäß Anspruch 1, worin die Loop-Sequenz komplementär zu wenigstens einem Teil einer Calcitonin-Nukleinsäuresequenz ist, die Methylierung unterliegt, wenn eine Zelle von einem normalen Zustand in einen kanzerösen Zustand übergeht.

13. Verfahren gemäß Anspruch 1, worin das Verfahren verwendet wird, um abnormal methylierte Gensequenzen in Prostatakrebsgewebe zu detektieren.

14. Verfahren gemäß irgendeinem vorangehenden Anspruch, in welchem die Hybridisierungsbedingung, die während der Hybridisierungsreaktion geändert wird, die Temperatur ist.

15. Verfahren gemäß irgendeinem vorangehenden Anspruch, in welchem die Stammsequenzen nicht an das Target-Gen hybridisieren und von ausreichend geringer Länger sind, dass nicht-spezifische Bindung zwischen dem Stamm und irgendeiner anderen Nukleinsäuresequenz in der Reaktionsmischung vermieden wird.

16. Verfahren gemäß irgendeinem vorangehenden Anspruch, in welchem die Stamm-Sequenzen eine Länge von ungefähr 4 bis 8 Nukleotide haben.

17. Verfahren gemäß irgendeinem der vorangehenden Ansprüche, in welchem wenigstens ein Cytosin in wenigstens einer der Stammsequenzen ein methylierter Cytosin-Rest ist.

## Revendications

1. Procédé de détection d'acides nucléiques méthylés comprenant les étapes de :
(i) mise en contact d'un échantillon d'acides nucléiques dont on suspecte qu'il contient des nucléotides méthylés, avec une séquence oligonucléotidique dans des conditions adéquates pour une hybridation d'acides nucléiques, ladite séquence oligonucléotidique étant **caractérisée en ce que**,
(a) elle comprend un premier radical avec un groupement fluorescent, une séquence en boucle ayant une région de nucléotides complémentaire au moins à une région de l'échantillon d'acide nucléique, laquelle région est susceptible d'être méthylée, et un second radical marqué avec un groupement extincteur qui est capable d'éteindre le groupement fluorophore quand il est à proximité spatiale du groupement fluorophore ; et
(b) que les nucléotides formant le premier radical sont capables de migrer à proximité spatiale des nucléotides formant le second radical quand la sonde est dissociée de l'échantillon d'acides nucléiques ;
(ii) altération des conditions d'hybridation de sorte que la sonde oligonucléotidique se dissocie de l'ADN non méthylé, mais reste hybridée à l'ADN méthylé ; et
(iii) mesure de la variation de la fluorescence.

2. Procédé selon la revendication 1, dans lequel quand les séquences oligonucléotidiques marquées se dissocient de l'échantillon d'acides nucléiques cible selon l'étape (ii), le premier et le second radical s'hybrident ensemble, provoquant l'extinction du groupement fluorophore.

3. Procédé selon la revendication 1 ou 2, dans lequel la séquence en boucle contient au moins 10 nucléotides.

4. Procédé selon la revendication 1 ou 2, dans lequel la séquence en boucle contient jusqu'à 35 nucléotides.

5. Procédé selon la revendication 1 ou 2, dans lequel la séquence en boucle contient entre 10 et 25 nucléotides.

6. Procédé selon la revendication 1, dans lequel la séquence en boucle contient au moins de 15 à 20 nucléotides.

7. Procédé selon la revendication 1, dans lequel la séquence en boucle est complémentaire d'une partie d'une séquence d'acides nucléiques qui subit la méthylation quand une cellule se transforme d'un état normal en un état cancéreux.

8. Procédé selon la revendication 1, dans lequel la séquence en boucle est complémentaire d'une partie de la séquence d'acide nucléique Myf-3 qui subit la méthylation quand une cellule se transforme d'un état normal en un état cancéreux.

9. Procédé selon la revendication 8, dans lequel la séquence oligonucléotidique marquée est complémentaire d'au moins l'une des séquences choisies dans le groupe constitué de :
(i) 
5′ GCG GCG ACT CCG ACG CGT CCA GCC CGC
GCT CC 3′
(ii) 
5′ TTA TAC CGC AGG CGG GCG AGC CGC GGG
CGC TCG CT 3′
(iii)

5′ CCG AGA GCC CTG CGG GGC CCG CCC TCC
TGC TGG CG 3′

10. Procédé selon la revendication 1, dans lequel la séquence en boucle est complémentaire d'une partie de la séquence d'acide nucléique gluthation-S-transférase-Π (pi) qui subit la méthylation quand une cellule se transforme d'un état normal en un état cancéreux.

11. Procédé selon la revendication 10, dans lequel la séquence oligonucléotidique marquée est complémentaire d'au moins l'une des séquences choisies dans le groupe constitué de :
(i) 
5′ CTC CAG CGA AGG CCT CGC GGC CTC CGA
GCC TTA TAA G 3′
(ii)

5′ GGG GAC GCG GGC CGC GCG TAC TCA CTG
GTG GCG A 3′

12. Procédé selon la revendication 1, dans lequel la séquence en boucle est complémentaire d'une partie de la séquence d'acide nucléique de la calcitonine qui subit la méthylation quand une cellule se transforme d'un état normal en un état cancéreux.

13. Procédé selon la revendication 1, dans lequel le procédé est utilisé pour détecter des séquences de gène anormalement méthylées dans des tissus du cancer de la prostate.

14. Procédé selon l'une quelconque des revendications précédentes, dans lequel la condition d'hybridation qui est altérée pendant la réaction d'hybridation, est la température de la réaction d'hybridation.

15. Procédé selon l'une quelconque des revendications précédentes, dans lequel les séquences de radicaux ne s'hybrident pas au gène cible, et sont d'une longueur suffisamment courte pour éviter la fixation non spécifique entre le radical et n'importe quelle autre séquence d'acides nucléiques dans le mélange réactionnel.

16. Procédé selon l'une quelconque des revendications précédentes, dans lequel les séquences de radicaux ont entre 4 et 8 nucléotides de long.

17. Procédé selon l'une quelconque des revendications précédentes, dans lequel au moins une cytosine dans au moins une séquence de radical, est un résidu de cytosine méthylé.
